# EUROPEAN PATENT SPECIFICATION

(11) **EP 3 310 398 B1**
(45) Date of publication and mention of the grant of the patent: **23.04.2025**
(21) Application number: 15730472.6
(22) Date of filing: 16.06.2015
(51) Int. Cl.: A61L 2/18, D06M 13/196, D06M 16/00

(54) **DAMP STORAGE OF CLEANING TEXTILES**
KLAMME AUFBEWAHRUNG VON REINIGUNGSTEXTILIEN
STOCKAGE HUMIDE DE TEXTILES DE NETTOYAGE

(43) Date of publication of application: 25.04.2018
(73) Proprietor: Ecolab USA Inc., St. Paul, MN 55102 (US); Leuchten, Elke, 42719 Solingen (DE); Hansen, Thomas, 41517 Grevenbroich (DE); Schepers, Freek, 40225 Düsseldorf (DE)
(72) Inventor: LEUCHTEN, Elke, 42719 Solingen (DE); HANSEN, Thomas, 41517 Grevenbroich (DE); SCHEPERS, Freek, 40597 Düsseldorf (DE)
(74) Representative: Michalski Hüttermann & Partner Patentanwälte mbB
(86) International application number: PCT/EP2015/063479
(87) International publication number: WO 2016/202366

(56) References cited:
- WO-A1-2012/099526
- WO-A1-95/04128
- WO-A1-95/17977
- WO-A2-2009/138888
- AU-A1- 2012 201 801
- US-A1- 2006 053 579

## Description

### Technical field of the invention

The invention relates generally to cleaning textiles and more specifically to the storage of cleaning textiles.

### Background of the invention

Surface cleaning is an important aspect of hygiene, in particular in sensitive areas such as hospitals or nursing homes. Usually different cleaning textiles are used for cleaning different surfaces. For example mops are used for cleaning floors, whereas scouring cloths are employed for cleaning tableware, furniture, sinks, showers or toilet bowls. On one hand, the cleaning textiles serve removing dirt and microbes from surfaces. On the other hand, cleaning textiles may not only spread microbes if they are not replaced often enough, but even before being used, as they provide a supreme substrate for microbial inhabitation and proliferation.

To prevent microbial inhabitation and proliferation on cleaning textiles, it is usually recommended to only store completely dried cleaning textiles after they were washed for their recycling. However, drying washed cleaning textiles is time consuming and energy consuming. Anyhow, damp storage of washed cleaning textiles should be avoided, because residual microbes in the cleaning textile can proliferate and cause mustiness, decay and malodour within a very few days.

US 2006/053579 A1 relates to method and apparatus for cleaning and/or disinfecting floor surfaces. A floor service trolley defines mop cloth container receptacles and a soiled mop cloth receptacle. Rectangular tapered mop cloth containers are supported by the container receptacle and define an upper sealing rim. A closure defines a seal recess containing a sealing member establishing a seal between the upper sealing rim and the closure preventing leakage of liquid from the container when it is inverted. Mop cloths are stacked within the container and are evenly pre-impregnated with cleaning solution by adding cleaning solution to the container of mop cloths, assembling the closure in sealed relation with the container and inverting the container for a sufficient period of time for saturation of the mop cloths by action of osmosis.

WO 95/04128 A1 relates to processes for the cleaning of textile by means of wet cleaning processes, in particular carried out in washing tubes. The present invention comprises a process for cleaning textile by means of wet cleaning in at least five phases, comprising a soaking phase, at least one sudsing phase, a rinsing phase, a bleaching phase, and a neutralization phase, in which in a second sudsing phase or in the rinsing phase a peroxy acid having at least 6 carbon atoms or a compound converted in situ into such a peroxy acid is added. The invention further comprises the use of peroxy acid having at least six carbon atoms or a compound giving in situ a peroxy acid having at least six carbon atoms for use as a disinfectant or bacteriostatic agent and/or as a bleaching agent in such a process.

WO 2012/099526 A1 relates to a container for impregnation of cleaning articles, the container comprising a watertight pouch made of a textile material or a plastic material The container is closable and is adapted, before being closed, to receive a number of cleaning textiles and also water or cleaning fluid, and, after being closed, to be turned upside down for impregnation of the cleaning textiles. The invention also relates to a cleaning system and method for impregnation of the cleaning textiles.

WO 2009/138888 A2 relates to a disinfectant wet wipe that contains a germicidal solution and a nonwoven web material is provided.

WO 95/17977 A1 relates to a method of cleaning beverage dispensing equipment, especially beer dispense lines, is disclosed, involving the use of peracetic acid as an active cleaning ingredient. Also described is a kit for such use.

AU 2012201801 A1 relates to sulfoperoxycarboxylic acid compounds, and methods for making and using them. The sulfoperoxycarboxylic compounds are storage stable, water soluble and have low to no odor. The compounds can be used as antimicrobials, and bleaching agents.

It is known to sterilize textiles upon washing them for further use. For example laid open publication BR 85 00 846 A teaches the use of an organic peracid, especially peracetic acid, as a bleach and disinfectant during commercial laundering of garments by means of an automatic or semiautomatic dispenser during the pre-soak, wash, or rinse stage of the washing cycle. In an example, 1,000 mg peracetic acid was added during the rinse of cold washed hospital garment, and no bacteria were detected at the end of the washing cycle.

With respect to cleaning textiles, it is known to perform the last rinse upon recycling mop heads in the presence of quaternary ammonium compounds to prevent mustiness and malodour during damp storage of the mop heads.

However, it turns out that disinfecting mop heads by supplying a quaternary ammonium compound to the last rinse affects appearance and durability of the mops. In addition, user compliance for the presence of quaternary ammonium compounds in cleaning textiles decreases due to reports wherein inadequate use of cleaning textiles being impregnated with quaternary ammonium compounds lead to the detection of residual amounts of quaternary ammonium compounds in foodstuff.

It was therefore an object of the present invention to provide an alternative for recycling cleaning textiles suitable for damp storage, which does not possess the drawbacks that were found to be associated with rinsing cleaning textiles in the presence of quaternary ammonium compounds.

The object is solved by a method and by cleaning textiles according to the independent claims. Preferred embodiments of the method and the cleaning textiles are subject matter of the dependent claims.

### SUMMERY OF THE INVENTION

In a first aspect, the invention provides a method for recycling cleaning textiles such that the proliferation of microbes on the cleaning textile upon damp storage of the cleaning textiles is prevented or at least inhibited.

In a second aspect, the invention provides the use of the cleaning textiles obtained by the method of the first aspect for cleaning surfaces.

### Detailed description

According to the first aspect, a method for recycling cleaning textiles is provided, wherein the proliferation of microbes on the cleaning textile is prevented or at least inhibited when the cleaning textile is stored in damp condition. The method for recycling a cleaning textile comprises the steps of:
- washing the cleaning textile;
- rinsing the cleaning textiles at least once, and
- spin-drying the cleaning textiles; and
- storing the spin-dried cleaning textile in damp condition;
wherein the last rinse is performed in the presence of an organic peracid supplied to the rinsing fluid.

In the method, the last rinse of the cleaning textile is performed in the presence of an organic peracid, preferably in the presence of peracetic acid.

The washing of the cleaning textiles may be performed at any suitable temperature, in any suitable washing machine and in the presence of any suitable laundry detergent. The various parameters and washing conditions are typically chosen - among other criteria - according to the type of textiles, the textile material, and the kind and degree of dirt, grime and/or contamination.

In an embodiment, the washing of the cleaning textile is performed in the presence of a disinfectant. The disinfectant may be selected from the group of disinfectants consisting of quaternary ammonium compounds and organic peracids. In an alternative and/or additional embodiment, the disinfectant in the washing step is peracetic acid. The disinfectant may be included in the laundry detergent or may be supplied separately to the washing machine before or during the washing step of the cleaning textile. The disinfectant is present in an effective amount during the washing step. For example, if the disinfectant is peracetic acid, the peracetic acid is supplied to the washer fluid in an amount of between about 50 mg/liter washer fluid to about 1,000 mg/liter.

Subsequent to the washing step(s), the cleaning textile is rinsed for removing residual washer fluid and residual amounts of laundry detergent. The cleaning textile is rinsed at least once. Preferably, the cleaning textile is rinsed at least two times. In a preferred embodiment, the cleaning textile is rinsed three times.

In the method of the invention, the last rinse is performed in the presence of an organic peracid, preferably in the presence of peracetic acid.

In an embodiment, the last rinse is performed in the presence of an amount of peracetic acid in that the peracetic acid is supplied to the rinsing fluid in an amount of at least 4.5 mg/liter rinsing fluid, preferably in an amount of at least 10 mg/liter rinsing fluid, more preferably in an amount of at least 20 mg/liter rinsing fluid. In an additional and/or alternative embodiment, the last rinse is performed in the presence of peracetic acid in that the peracetic acid is supplied to the rinsing fluid in an amount of less than 1,000 mg/liter rinsing fluid, preferably in an amount of less than 100 mg/liter rinsing fluid, more preferably in an amount of less than 25 mg/liter rinsing fluid. In additional and/or alternative embodiments, the last rinse is performed in the presence an amount of peracetic acid in that the peracetic acid is supplied to the rinsing fluid in an amount of about 4.5 mg/liter rinsing fluid, in that the peracetic acid is supplied to the rinsing fluid in the presence of an amount of about 13.5 mg/liter rinsing fluid, or in that the peracetic acid is supplied to the rinsing fluid in an amount of about 22.5 mg/liter rinsing fluid.

Peracetic acid is an organic compound represented by the formula CH₃CO₃H. Peracetic acid is formed upon treatment of acetic acid with hydrogen peroxide, with the equilibrium constant dependent on the concentrations and conditions of the reaction.

In an alternative and/or additional embodiment, the rinsing fluid further contains one or more compounds selected from the group consisting of acetic acid, hydrogen peroxide and a phosphonate, preferably 1-hydroxyethylidene-1,1-diphosphonic acid (HEDP). In an embodiment, the rinsing fluid of the last rinse step does not contain any further compounds, in particular no additional disinfectants. Thus, according to another and/or additional embodiment, the rinsing fluid of the last rinse is an aqueous solution consisting of water, acetic acid, peracetic acid, hydrogen peroxide and 1-hydroxyethylidene-1,1-diphosphonic acid. In an embodiment, the aqueous solution consisting of water, acetic acid, peracetic acid, hydrogen peroxide and 1-hydroxyethylidene-1,1-diphosphonic acid comprises from about 4.5 mg/liter to about 22.5 mg/liter peracetic acid, preferably about 13.5 mg/liter peracetic acid, from about 23 mg/liter to about 115 mg/liter hydrogen peroxide, preferably about 69 mg/liter, and from about 0.6 mg/liter to about 3.0 mg/liter HEDP, preferably about 1.8 mg/liter HEDP, with respect to the rinsing fluid.

Subsequent to the last rinse, the cleaning textiles are spin-dried to become damp.

The term "damp" or "damp textile" refers to a textile comprising a residual amount of moisture, wherein the residual amount of moisture can hardly be manually squeezed out of the textile. The amount of residual moisture in a "damp textile" may vary upon the textile's material. However, "damp textile" in the context of the instant disclosure refers to a moisture content of less than about 32 %-wt., preferably to a moisture content of less than about 25 %-wt., more preferably to a moisture content of less than about 17 wt.-%, and most preferably to a moisture content of about 14 %-wt., with reference to the weight of the dry cleaning textile.

In an additional and/or alternative embodiment, the cleaning textile is selected from the group consisting of mops, mop heads, swabs, wiping cloths and floor cloths.

The damp cleaning textiles may be subjected to damp storage, i.e. they are stored such that the residual moisture in the cleaning textile can not evaporate, and the cleaning textile does not become dry. For damp storage, the damp cleaning textiles may be put in a plastic container provided with a tightly closing lid, or they may be sealed in a plastic bag. The damp cleaning textiles may be subjected to damp storage for at least up to 72 hours at room temperature (i.e. at 21°C to 23°C) without any detectable malodour, decay or mustiness.

The method according to the invention provides for damp storage of the cleaning textiles obtained from said method for at least up to 72 hour. The method according to the invention is advantageous as it is an environmentally friendly method, because peracetic acid disintegrates into nontoxic compounds. User compliance of residual amounts of peracetic acid in cleaning textiles is far better than user compliance of residual amounts of quaternary ammonium compounds in cleaning textiles.

In addition, it was surprisingly found that rinsing cleaning textiles in the presence of peracetic acid neither affects durability nor performance of the cleaning textile, not even when subjected multiple times to the method of the invention.

The method for recycling cleaning textiles may also be considered as a method of improving damp storage of cleaning textiles, because recycled cleaning textiles do not need to be dried to the utmost extend in order to avoid malodour, decay and/or mustiness, but may be stored in damp condition for at least 72 hours without possessing malodour, decay and/or mustiness.

According to the second aspect, the invention is directed to the use of a cleaning textile that is obtainable by a method as described herein before. The cleaning textile is a damp cleaning textile.

In an additional and/or alternative embodiment, the cleaning textile is a recycled cleaning textile. A recycled cleaning textile is a cleaning textile that was already used for cleaning purposes, and that has been washed after its use such that it may be used again.

In an additional and/or alternative embodiment, the cleaning textile has a moisture content of less than about 32 %-wt., preferably to a moisture content of less than about 25 %-wt., more preferably to a moisture content of less than about 17 %-wt, and most preferably to a moisture content of about 14 %-wt.

In an additional and/or alternative embodiment, the moisture of the cleaning textile comprises an organic peracid, preferably peracetic acid.

In an additional and/or alternative embodiment, the moisture comprises peracetic acid in an amount of at least 4.5 mg/liter moisture, preferably in an amount of at least 10 mg/liter moisture, more preferably in an amount of at least 20 mg/liter moisture.

In an additional and/or alternative embodiment, the moisture comprises peracetic acid in an amount of less than 1,000 mg/liter moisture, preferably in an amount of less than 100 mg/liter moisture, more preferably in an amount of less than 25 mg/liter moisture.

In an additional and/or alternative embodiment, the moisture comprises peracetic acid in an amount of about 4.5 mg/liter moisture, in an amount of about 13.5 mg/liter moisture, or in an amount of about 22.5 mg/liter moisture.

In an additional and/or alternative embodiment, the moisture may further contain one or more compounds selected from the group consisting of acetic acid, hydrogen peroxide and a phosphonate, preferably 1-hydroxyethylidene-1,1-diphosphonic acid (HEDP). In an embodiment, the moisture does not contain any further compounds, in particular no additional disinfectants. Thus, according to another and/or additional embodiment, the moisture is an aqueous solution consisting of water, acetic acid, peracetic acid, hydrogen peroxide and 1-hydroxyethylidene-1,1-diphosphonic acid. In an embodiment, the aqueous solution consisting of water, acetic acid, peracetic acid, hydrogen peroxide and 1-hydroxyethylidene-1,1-diphosphonic acid comprises from about 4.5 mg/liter to about 22.5 mg/liter peracetic acid, preferably about 13.5 mg/liter peracetic acid, from about 23 mg/liter to about 115 mg/liter hydrogen peroxide, preferably about 69 mg/liter, and from about 0.6 mg/liter to about 3.0 mg/liter HEDP, preferably about 1.8 mg/liter HEDP, with respect to the moisture.

In an additional and/or alternative embodiment, the damp cleaning textile is present in a tightly closed or sealed container, preferably plastic container, or bag, preferably plastic bag, such that the cleaning textile remains damp for a period of at least 72 hours at room temperature.

According to the second aspect, the invention provides the use of the cleaning textiles obtained by the method of the first aspect for cleaning purposes, preferably for cleaning surfaces, especially for cleaning surfaces in hygienically sensitive areas such as hospitals, medical practices, nursing homes, kindergarten or sanitary facilities.

The present invention will be further described with respect to particular embodiments, but the invention is not limited thereto but only by the claims.

The terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim.

The claims following the detailed description are hereby expressly incorporated into this detailed description, with each claim standing on its own as a separate embodiment of this invention.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

### Example 1

Three different mop heads were chosen as test objects for investigating the bacteriostatic or antibacterial effect of a peracetic acid supply in the last rinse on recycled cleaning textiles.

Test object 1 (TO1) were mop heads made of 50% polyester and 50% cotton. The inner portion of the mop head comprised tufted loops whereas the edges comprised sewn fringes.

Test object 2 (TO2) were mop heads consisting of a polyester pile fabric. The pile consisted of 20% polyester yarn and 80 % polyester microfibers.

Test object 3 (TO3) were mop heads comprising a pile fabric made of 78% polyester microfibers and 22% polyamide fibers.

For testing, the different test objects were washed in a disinfectant washing procedure. Subsequent to the washing cycle, the test objects were rinsed three times and different amounts of peracetic acid were supplied to the last rinse. At the end of the washing process, the mop heads were spin-dried at 1,000 rpm. Thereafter, the damp mop heads were stored tightly sealed in plastic bags for up to 72 hours at room temperature. Samples of each test object were removed at time intervals as indicated herein after and further assessed for their microbial load, their appearance, and their performance.

### Description of the test method:

For all three test objects, 6 kg mop heads of each of the test objects were loaded into a professional washing machine (Miele Professional WS5071 Mop-Profi) and washed for 15 minutes at 60°C using 1.5 ml Ecobrite^{®} Magic Emulsion and 2 ml/l Ozonit^{®}. The liquor ratio was 1:5.

Ozonit^{®} is an aqueous solution consisting of 4.5 wt.-% per acetic acid, 23 wt.-% H₂O₂ and 0.6 wt.-% 1-hydroxyethylidene-1,1-diphosphonic acid.

Immediately after the washing cycle, the test objects were subjected to two rinse cycles with cold water. The liquor ratio was 1:5.

A third rinse cycle followed in cold water too. The liquor ratio was again 1:5, and the rinsing water was supplemented with 0.1 ml Ozonit^{®}/liter rinsing water, 0.3 ml Ozonit^{®}/liter rinsing water, or 0.5 ml Ozonit^{®}/liter rinsing water.

Samples of the different test objects were stored in tightly closed plastic bags at room temperature for 24 hours or 72 hour before the bacterial load of representative commensurate test pieces (5 cm x 5 cm) was determined by extracting the bacteria from said test pieces using 100 ml of a recovery solution containing Tween, lecithin, histidine and sodium thiosulphate. The bacterial load was determined in that viable counts were revealed by dilution series performed as spread method.

The results are shown in tables 1 to 3 herein after.

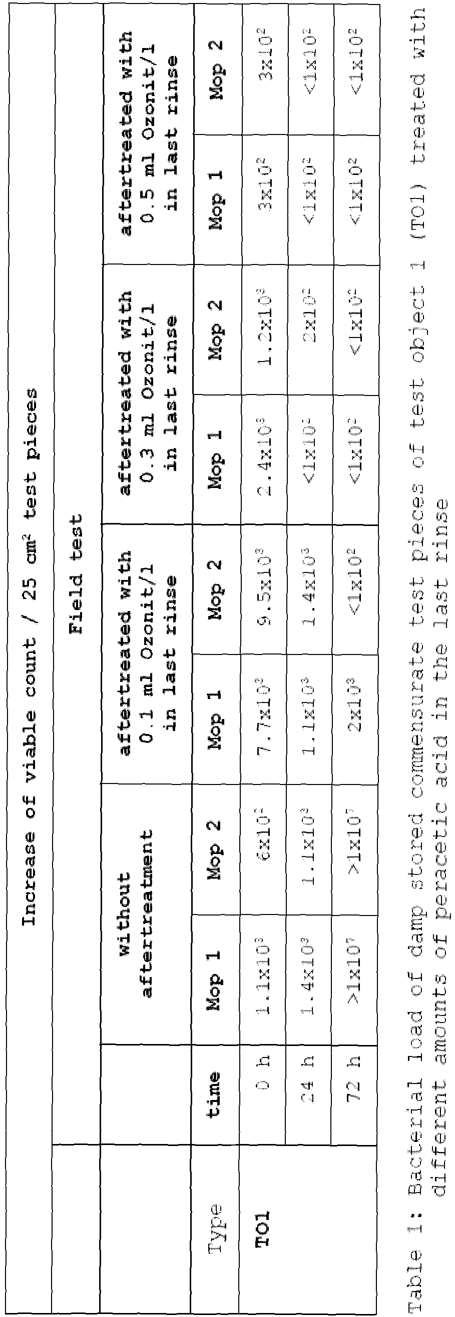

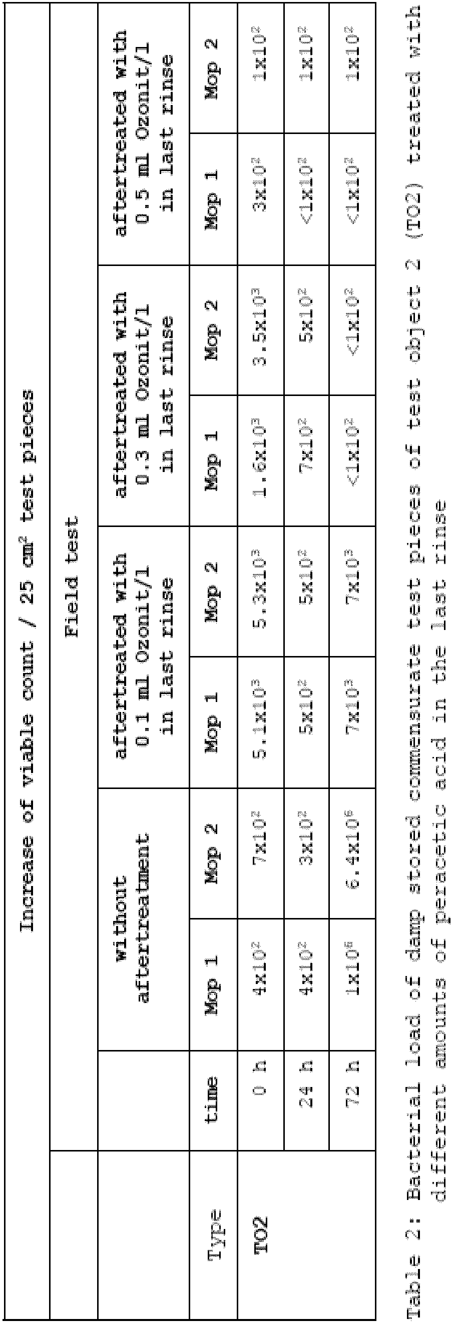

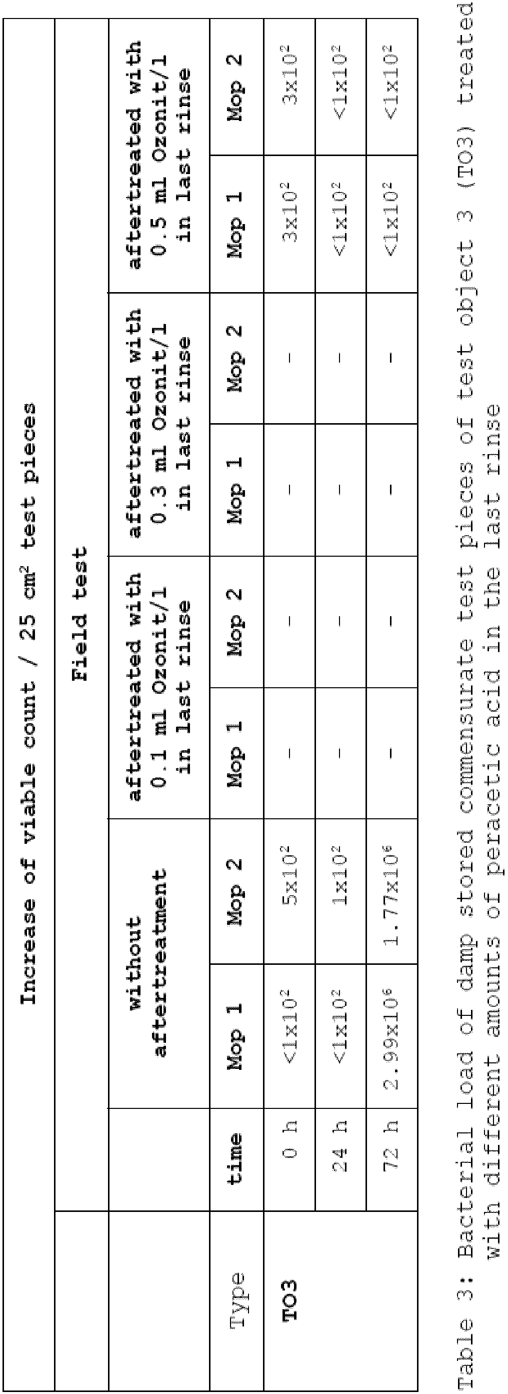

These data reveal that the growth of bacteria on damp stored cleaning textiles can be prevents for at least 72 hours if treated with peracetic acid in the last rinse of the recycling procedure. Even with an amount of as little as 0.1 ml Ozonit^{®} per liter rinsing fluid (which corresponds to about 4.5 mg peracetic acid per liter rinsing fluid, the number of viable counts remained stable during damp storage. Using larger amounts of peracetic acid in the last rinse show a bactericidal effect of peracetic acid during damp storage of the mop heads.

### Example 2

For assessing the effect of peracetic acid in the last rinse on the appearance and performance of cleaning textiles, the different test objects as described in Example 1 were washed, rinsed and spin-dried as described in Example 1 for 25 times or 50 times with a 2 hour damp storage in the washing machine after each washing/rinsing/spin-drying cycle before being assessed. In each cycle 0.5 ml Ozonit^{®} was supplied to each liter rinsing fluid in the last of three rinsing steps.

First, the test objects were assessed for optical impression, but there was no optical difference between the test objects washed 25 times and the corresponding test objects washed 50 times with respect to colour change, greying or yellowing.

Second, the test objects were assessed for durability. The effect of 22.5 mg peracetic acid per liter in the last rinse on the dry weight of different mop heads is shown in table 4:

**Table 4: Weight loss of mop heads upon the presence of peracetic acid in the last rinse.**

| | **TO1** | **TO2** | **TO3** |
|---|---|---|---|
| **50 washing cycles without Ozonit in the last rinse** | | | |
| Mop weight new (g) | 166 | 71 | 89 |
| Mop weight after 50 washing cycles (g) | 163 | 71 | 89 |

| **50 washing cycles with Ozonit in the last rinse** | | | |
|---|---|---|---|
| Mop weight new (g) | 166 | 71 | 89 |
| Mop weight after 50 washing cycles (g) | 165 | 71 | 89 |

The results as shown in table 4 demonstrate that peracetic acid in the last rinse does not affect the dry weight of different mop heads.

Third, the test objects were assessed for performance. The effect of 22.5 mg peracetic acid per liter in the last rinse on the water absorption capacity of different mop heads is shown in table 5:

**Table 5: Absorption capacity for water of mop heads upon the presence of peracetic acid in the last rinse.**

| | **TO1** | **TO2** | **TO3** |
|---|---|---|---|
| **50 washing cycles without Ozonit in the last rinse** | | | |
| Water absorption (g water / mop) | 470 | 228 | 226 |

| **50 washing cycles with Ozonit in the last rinse** | | | |
|---|---|---|---|
| Water absorption (g water / mop) | 473 | 231 | 237 |

The results shown in Table 5 reveal that the addition of 22.5 mg peracetic acid per liter rinsing fluid in the last rinse did not affect the water absorption capacity of different mop heads. Thus, the presence of peracetic acid in the last rinse of mop heads did not affect their performance as measured by means of their water absorption capacity.

### Example 3

For further investigating the effect of peracetic acid in the last rinse on cleaning textiles, standard washing control strips were used instead of the different test objects for assaying the effect of peracetic acid in the last rinse. Standard cotton washing control strips (40 cm x 100 cm) were subjected to 50 washing cycles, each cycle comprising three consecutive rinse steps subsequent to the washing step, wherein the last of each third rinse step was performed either in the absence or in the presence of 0.5 ml Ozonit^{®} per liter rinsing fluid (corresponds to 22.5 mg/ml peracetic acid per liter rinsing fluid) .

The washing control strips were than analyzed and evaluated by the wkf - Cleaning Technology Institute e.V. in Krefeld, Germany. The results are summarized in table 6.

**Table 6: Evaluation of washing control strips**

| | with peracetic acid | | without peracetic acid | |
|---|---|---|---|---|
| | probe 1 | probe 2 | probe 3 | probe 4 |
| WG-value | 193 ± 4 | 204 ± 4 | 204 ± 2 | 203 ± 3 |
| Y-value | 89 ± 0.1 | 90 ± 0.3 | 90 ± 0.2 | 90 ± 0.2 |
| tensile strength (daN) | 87 ± 3 | 88 ± 3 | 87 ± 3 | 86 ± 2 |
| loss of tensile strength (%) | 5 | 4 | 5 | 7 |
| damage factor (s) | 0 | 0 | 0.1 | 0 |
| glowing ash (%) | 0.5 | 0.8 | 0.5 | 0.7 |

The WG-value determines the brightness according to GANZ. The brightness according to GANZ describes the impression of brightness perceived by the human eye. The WG-value is determined by the spectral process according to DIN 5033 part 4 or by the tristimulus method according to DIN 5033 part 6 using illumination by xenon lamps and sufficient approximation to normal light of kind D₆₅. The brightness was calculated utilizing the CIE color coordinates. The equation for calculating the brightness according to GNAZ is W = (D * Y) + (P * x) + (Q * y) + C, wherein Y is the measure for brightness, x and y are chromaticity coordinates of the probe. D, P and Q are parameters effecting the white appearance.

The Y-value is the basic white value representing the white appearance of the textile after filtering off the UV portion of the light source and turning off the brightening effect. Determining the Y-value permits evaluation of whether washing caused graying of the fabric.

The loss of tensile strength is provided as percentage of the tensile strength of identical washing control strips measured prior to the washing cycles and was always measured in warp direction.

The damage factor indicates the chemical damage of the fibers in the textile by means of chemical decay.

The value for glowing ash indicates an accumulation of inorganic compounds on the fabric. For measuring the glowing ash, the textile was shed and the resulting ash was weighed analytically.

The results obtained from analyzing the washing control stripes reveal that the presence of 22.5 mg peracetic acid per liter rinsing fluid in the last rinse neither affected durability nor appearance of the fabric, because none of the properties of the washing control strips that were analyzed differed between washing control strips that were rinsed in the presence of peracetic acid and the corresponding washing control strips that were rinsed in the absence of peracetic acid.

From the foregoing detailed description and examples, it will be evident that modifications and variations can be made to the compositions and methods. It is intended that all modifications made to the invention without departing from the scope of the invention come within the scope of the appended claims.

## Claims

1. A method for recycling a cleaning textile, the method comprising the steps of:
- washing the cleaning textile;
- rinsing the cleaning textile at least one time;
- spin-drying the cleaning textile; and
- storing the spin-dried cleaning textile in damp condition;
wherein the last rinse is performed in the presence of an organic peracid supplied to the rinsing fluid.

2. The method according to claim 1, wherein the organic peracid is peracetic acid.

3. The method according to claim 1 or 2, wherein the peracetic acid is supplied to the rinsing fluid in an amount of at least 4.5 mg/liter rinsing fluid, preferably in an amount of at least 10 mg/liter rinsing fluid, more preferably in an amount of at least 20 mg/liter rinsing fluid.

4. The method according to any one of claims 1 to 3, wherein the peracetic acid is supplied to the rinsing fluid in an amount of less than 1,000 mg/liter rinsing fluid, preferably in an amount of less than 100 mg/liter rinsing fluid, more preferably in an amount of less than 25 mg/liter rinsing fluid.

5. The method according to any one of claims 1 to 4, wherein the cleaning textile is rinsed at least two times, preferably three times.

6. The method according to any one of claims 1 to 3, wherein the cleaning textile is spin-dried to a moisture content of less than about 32 %-wt., preferably to a moisture content of less than about 25 %-wt., more preferably to a moisture content of less than about 17 %-wt., and most preferably to a moisture content of about 14 %-wt.

7. The method according to any one of claims 1 to 6, wherein the cleaning textile is selected from the group consisting of mops, mop heads, swabs, wiping cloths and floor cloths.

8. Use of a cleaning textile obtained by a method of claims 1 to 7 for cleaning surfaces, comprising the method steps according to any of claims 1 to 7.

## Patentansprüche

1. Verfahren zum Wiederverwerten eines Reinigungstextils, das Verfahren umfassend die Schritte:
- Waschen des Reinigungstextils;
- Spülen des Reinigungstextils mindestens ein Mal;
- Schleudern des Reinigungstextils; und
- Lagern des geschleuderten Reinigungstextils in feuchtem Zustand;
wobei die letzte Spülung in der Gegenwart einer organischen Persäure durchgeführt wird, die dem Spülfluid zugeführt wird.

2. Verfahren nach Anspruch 1, wobei die organische Persäure Peroxyessigsäure ist.

3. Verfahren nach Anspruch 1 oder 2, wobei die Peroxyessigsäure dem Spülfluid in einer Menge von mindestens 4,5 mg/Liter Spülfluid, vorzugsweise in einer Menge von mindestens 10 mg/Liter Spülfluid, mehr bevorzugt in einer Menge von mindestens 20 mg/Liter Spülfluid, zugeführt wird.

4. Verfahren nach einem der Ansprüche 1 bis 3, wobei die Peroxyessigsäure dem Spülfluid in einer Menge von weniger als 1.000 mg/Liter Spülfluid, vorzugsweise in einer Menge von weniger als 100 mg/Liter Spülfluid, mehr bevorzugt in einer Menge von weniger als 25 mg/Liter Spülfluid, zugeführt wird.

5. Verfahren nach einem der Ansprüche 1 bis 4, wobei das Reinigungstextil mindestens zwei Mal, vorzugsweise drei Mal, gespült wird.

6. Verfahren nach einem der Ansprüche 1 bis 3, wobei das Reinigungstextil auf einen Feuchtigkeitsgehalt von weniger als etwa 32 Gew.-%., vorzugsweise auf einen Feuchtigkeitsgehalt von weniger als etwa 25 Gew.-%, mehr bevorzugt auf einen Feuchtigkeitsgehalt von weniger als etwa 17 Gew.-% und am meisten bevorzugt auf einen Feuchtigkeitsgehalt von etwa 14 Gew.-%, geschleudert wird.

7. Verfahren nach einem der Ansprüche 1 bis 6, wobei das Reinigungstextil aus der Gruppe ausgewählt ist, bestehend aus Mopps, Moppköpfen, Lappen, Wischtüchern und Bodenwischtüchern.

8. Verwendung eines Reinigungstextils, das durch ein Verfahren nach den Ansprüche 1 bis 7 erhalten wird, zum Reinigen von Oberflächen, umfassend die Verfahrensschritte nach einem der Ansprüche 1 bis 7.

## Revendications

1. Procédé de recyclage d'un textile de nettoyage, le procédé comprenant les étapes consistant à :
- laver le textile de nettoyage ;
- rincer le textile de nettoyage au moins une fois ;
- l'essorage du textile de nettoyage ; et
- stocker le textile de nettoyage essoré à l'état humide ;
dans lequel le dernier rinçage est effectué en présence d'un peracide organique fourni au liquide de rinçage.

2. Procédé selon la revendication 1, dans lequel le peracide organique est l'acide peracétique.

3. Procédé selon la revendication 1 ou 2, dans lequel l'acide peracétique est fourni au liquide de rinçage en une quantité d'au moins 4,5 mg/litre de liquide de rinçage, de préférence en une quantité d'au moins 10 mg/litre de liquide de rinçage, plus préférablement en une quantité d'au moins 20 mg/litre de liquide de rinçage.

4. Procédé selon l'une quelconque des revendications 1 à 3, dans lequel l'acide peracétique est fourni au liquide de rinçage en une quantité inférieure à 1 000 mg/litre de liquide de rinçage, de préférence en une quantité inférieure à 100 mg/litre de liquide de rinçage, plus préférablement en une quantité inférieure à 25 mg/litre de liquide de rinçage.

5. Procédé selon l'une quelconque des revendications 1 à 4, dans lequel le textile de nettoyage est rincé au moins deux fois, de préférence trois fois.

6. Procédé selon l'une quelconque des revendications 1 à 3, dans lequel le textile de nettoyage est essoré jusqu'à un taux d'humidité inférieur à environ 32 % en poids, de préférence jusqu'à un taux d'humidité inférieur à environ 25 % en poids, plus préférablement jusqu'à un taux d'humidité inférieur à environ 17 % en poids, et encore plus préférablement jusqu'à un taux d'humidité d'environ 14 % en poids.

7. Procédé selon l'une quelconque des revendications 1 à 6, dans lequel le textile de nettoyage est choisi dans le groupe constitué de serpillières, têtes de serpillière, tampons, chiffons d'essuyage et serpillières.

8. Utilisation d'un textile de nettoyage obtenu par un procédé selon les revendications 1 à 7 pour le nettoyage de surfaces, comprenant les étapes de procédé selon l'une quelconque des revendications 1 à 7.
